## Europäisches Patentamt

## European Patent Office

## Office européen des brevets

(19)

(11) Veröffentlichungsnummer: **0 049 812**

**A1**

(12) # EUROPÄISCHE PATENTANMELDUNG

(21) Anmeldenummer: **81107745.2**

(22) Anmeldetag: **29.09.81**

(51) Int. Cl.³: **A 61 N 1/08**
**A 61 N 1/36**

(30) Priorität: **10.10.80 DE 3038856**

(43) Veröffentlichungstag der Anmeldung:
**21.04.82 Patentblatt 82/16**

(84) Benannte Vertragsstaaten:
**FR GB IT NL**

(71) Anmelder: **SIEMENS AKTIENGESELLSCHAFT Berlin und München**
**Postfach 22 02 61**
**D-8000 München 22(DE)**

(72) Erfinder: **Elmqvist, Häkan, Dr.**
**Sunnerdalsvägen 7**
**S-16 138 Bromma(SE)**

(54) **Herzschrittmacher.**

(57) Von der Herstellung bis hin zur Anwendung eines Herzschrittmachers fallen eine Vielzahl von administrativen und diagnostischen Daten an, die zu unterschiedlichsten Zeiten z. B. einem behandelnden Arzt vorliegen müssen, um eine Beurteilung der Herzschrittmacherfunktionen und seine Wirkungen auf einen Patienten vornehmen zu können. Bisher wurden zum Sammeln der Daten eine Reihe unterschiedlicher Datenträger, wie Patientenkarteien, Testkarten, Formulare und andere Dokumente verwendet. Um den Arbeitsaufwand für das Sammeln zu reduzieren und gleichzeitig alle Daten sicher und geschlossen darstellen zu können, ist erfindungsgemäß ein Herzschrittmacher (1) mit einem Speicher (3) für Daten, die nicht direkt mit der Funktion des Herzschrittmachers zu tun haben, Mitteln (6) zum kontaktlosen Empfangen und/oder Übertragen von Daten an bzw. von einer äußeren Apparatur (Peripheriegerät 10) sowie eine Steuereinheit (4) für den Datentransport vorgesehen. Dadurch übernimmt der Herzschrittmacher (1) die administrativen Routinen von der Herstellung bis zur Anwendung und zumindest auch einen Teil der diagnostischen Werte selbst. In einer vorteilhaften Weiterbildung ist ein Meßwerterfassungsglied (7) und ein Datenaufbereitungsglied (8) vorgesehen, um speziell diagnostische Daten direkt intern abfragen zu können.

FIG 2

PERIPHERIEEINHEIT FÜR LANGZEITREGISTRIERUNG

SIEMENS AKTIENGESELLSCHAFT          Unser Zeichen
Berlin und München                  VPA 80 P 5961  E

Herzschrittmacher

Die Erfindung betrifft einen Herzschrittmacher, insbesondere einen implantierbaren. Sie ist unabhängig von der Art des verwendeten Herzschrittmacheres. So kann es sich um solche mit fester Impulsfrequenz, um nach dem "Deman-Prinzip" arbeitende oder auch programmierbare handeln.

Von der Herstellung über den Vertrieb und die Anwendung von Herzschrittmachern bis hin zum Ende einer Herzschrittmacherbehandlung fallen eine Menge administrative Daten an, die gesammelt und bearbeitet werden müssen. Solche Daten können z. B. das Herzschrittmachermodell, seine Seriennummer, die Kunden- und Patientenidentität, das Datum für die Implantation und Explantation und eventuelle Angaben über Zeitpunkt und Art einer möglichen Umprogrammierung und viele andere sein. Heutzutage werden diese administrativen Aufgaben mit Hilfe von Dokumenten, wie z. B. Konteroll-Listen bei der Produktion, Endtestkarten, Patientenkarteien und anderen Formularen gelöst. Dieses System ist sehr arbeitsaufwendig. Darüber hinaus besteht die Gefahr, daß sich beim Sammeln dieser Vielzahl von Daten an unterschiedlichsten Stellen und bei dem Umgang mit diesem Material Fehler einschleichen.

Eine andere Schwierigkeit für den behandelnden Arzt bei einer Herzschrittmacher-Behandlung besteht darin, zu beurteilen, wie der Herzschrittmacher funktioniert und ob sämtliche Funktionen korrekt ausgeführt werden. Auf der einen Seite geht es dabei um eventuelle technische Fehler des Schrittmachers selbst, auf der anderen Seite um die Wechselwirkung zwischen dem Schrittmacher,

Gdl 5 Un / 09.10.1980

den Elektroden und dem zu stimulierenden Herzen. Weiterhin interessiert auch die Reaktion des Patienten auf eine Herzschrittmacher-Behandlung.

Um sich über all diese Fragen Klarheit zu verschaffen, führt der behandelnde Arzt u. a. eine Amnese durch, d. h. er befragt den Patienten. Des weiteren nimmt er ein EKG auf, macht eine Pulsfotoanalyse usw. Bei gewissen Herzschrittmachertypen ist es heutzutage bereits möglich, Parameter wie den Stromverbrauch, die Elektrodenimpedanz und auch ein intrakardiales EKG zu messen. Alle diese zuletzt aufgeführten Angaben kann man unter dem Sammelbegriff "diagnostische Daten" zusammenfassen. Ebenso wie bei den administrativen ist das Einsammeln der diagnostischen Daten aufwendig und führt oft zu einer unsicheren Beurteilung.

Der vorliegenden Erfindung liegt die Aufgabe zugrunde, bei einem Herzschrittmacher der eingangs genannten Art den Arbeitsaufwand für das Sammeln und Bearbeiten der erforderlichen Daten erheblich zu reduzieren und den Umgang mit diesen Daten sicherer zu machen.

Diese Aufgabe wird erfindungsgemäß durch einen Speicher für Daten, die nicht direkt mit der Funktion des Herzschrittmachers zu tun haben, Mittel zum kontaktlosen Empfangen und/oder Überführen von Daten an bzw. von einer äußeren Apparatur (Peripheriegerät) sowie einer Steuereinheit für den Datenfluß gelöst. Der Speicher register kann dabei zur Aufnahme sowohl der administrativen als auch zur Aufnahme von diagnostischen Daten oder zumindest einem Teil dieser anfallenden Daten dienen.

In einer vorteilhaften Weiterbildung der Erfindung ist vorgesehen, daß ein Meßwerterfassungsglied im Herzschrittmacher selbst angeordnet ist, von dem aus Daten, gesteuert über die Steuereinheit, vorgewählten Plätzen des Speichers zuführbar sind. Weiterhin kann auch noch ein Datenaufbereitungsglied vorgesehen sein, mit dessen Hilfe die anfallende Datenmenge für eine platzsparende Speicherung verdichtet oder vorausgewertet werden kann.

Eine weitere wesentliche Vereinfachung für einen behandelnden Arzt ergibt sich dann, wenn die so gesammelten und gespeicherten administrativen sowohl als diagnostischen Daten in einer einfachen und übersichtlichen Form dargestellt werden können. Das Peripheriegerät kann zu diesem Zweck mit einer Datenausgabeeinheit, beispielsweise einem Bildschirm oder einem Schreiber versehen sein. Durch eine klare und übersichtliche Darstellung sämtlicher,teilweise auch aufbereiteter Daten wird die Beurteilung über den Einsatz eines Herzschrittmachers in einem Patienten durch den behandelnden Arzt wesentlich verbessert.

Eine Erweiterung der Datenspeicherung wird dadurch erreicht, daß das Peripheriegerät auch eine Dateneingabeeinheit aufweist, mit deren Hilfe der Inhalt des Speichers im Herzschrittmacher geändert werden kann.

Bei hohem Speicherbedarf kann unter Umständen auch in dem Peripheriegerät ein zusätzlicher Speicher vorgesehen sein. Damit gelingt es, die Abmessungen des Herzschrittmachers und damit die Belastung für den Organismus des Patienten kleinzuhalten.

Über einen Zeitgeber im Herzschrittmacher und/oder dem Peripheriegerät ist es möglich, bestimmte Zeiten für Arbeitsabläufe im Speicher oder zwischen diesen und dem Peripheriegerät langfristig festzulegen und vorher zu speichern.

Zusammenfassend läßt sich sagen, daß der erfindungsgemäße Herzschrittmacher alle oder zumindest einen Teil der administrativen Routinen von der·Herzschrittmacher-Herstellung bis zur Anwendung und auch die diagnostischen Angaben und Meßwerte selbst übernimmt.

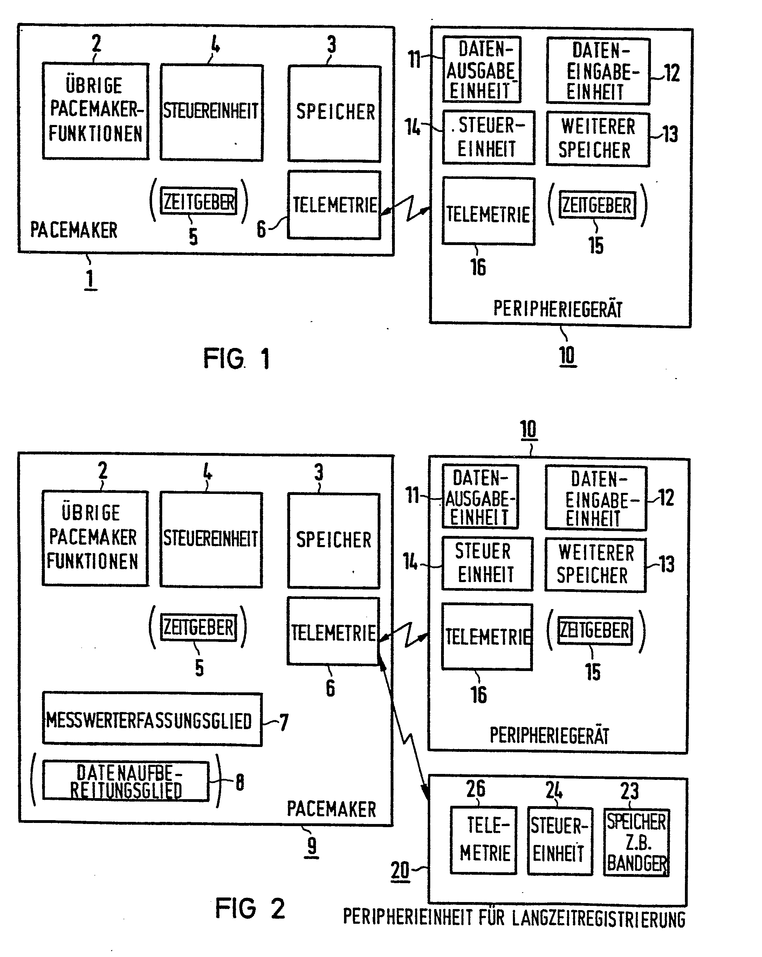

Weitere Vorteile des erfindungsgemäßen Herzschrittmachers ergeben sich aus den Unteransprüchen allein oder im Zusammenhang mit den nachfolgend anhand von zwei Figuren beschriebenen Ausführungsbeispielen. Dabei zeigt Fig. 1 in einem schematischen Blockschaltbild einen ersten Herzschrittmacher nach der Erfindung mit zugehörigem Peripheriegerät.
Fig. 2 zeigt eine erweiterte Ausführungsform dieses Herzschrittmachers und ein zusätzliches Peripheriegerät.

In Fig. 1 ist mit 1 der Herzschrittmacher bezeichnet. In dem Block 2 sollen sich alle herkömmlichen Elemente des verwendeten Herzschrittmachers befinden, wie beispielsweise der Generator zur Erzeugung der Stimulierungsimpulse, Einstellglieder für die Impulsamplitude, die Impulsfrequenz, Erkennungsglieder für die R-Zacke des Herzschlages und ähnliches. Mit 3 ist der erfindungsgemäße herzschrittmachereigene zusätzliche Speicher bezeichnet, in dem die administrativen und diagnostischen Daten gelagert werden können. Eine Steuereinheit 4 sorgt für die richtige Lagerung der Daten oder das Herausholen dieser Daten aus dem entsprechenden Speicherplatz zu einem vorgewählten Zeitpunkt. Mittels eines Zeitgebers 5 können zusätzlich Zeitpunkte für gewisse Arbeitsabläufe festgelegt werden. Mittel zum Empfangen oder Übertragen der notwendigen Daten sind mit 6 bezeichnet. Die als Peripheriegerät bezeichnete äußere Apparatur 10 enthält im vorliegenden Ausführungsbeispiel eine Datenausgabeeinheit 11, eine Dateneingabeeinheit 12, einen weiteren Speicher 13, eine Steuereinheit 14, einen Zeitgeber 15 und Mittel 16 für den Datentransport. Als Datenausgabeeinheit 11 kann z. B. ein Bildschirm oder ein Schreiber dienen. Als Dateneingabeeinheit kann z. B. eine Tastatur oder ein Kartenleser verwendet werden. Bei den Mitteln 6, 16 zur Datenübertragung zwischen Herzschrittmacher 1
und Peripheriegerät 10 kann man sich in bekannter Weise
der Fernmessung bedienen.

Um eine fehlerhafte bzw. ungewollte Lagerung von Daten
in dem Speicher 3 zu vermeiden, kann dieser zumindest
teilweise so ausgeführt sein, daß sein Inhalt durch
das Peripheriegerät 10 nicht verändert werden kann oder
daß zum Verändern ein ganz spezielles Peripheriegerät
erforderlich ist.

Bereits bei der Fertigung eines derartigen Herzschrittmachers 1 werden in den Speicher 3 gewisse  Daten eingegeben. Solche Daten können z. B. die Modellbezeichnung
des Herzschrittmachers, seine Seriennummer, gewisse
Parameter wie die Impulsamplitude, die Empffindlichkeit
und anderes sein. Diese Daten können anschließend jederzeit
abgelesen werden, beispielsweise bei der Auslieferung
des Herzschrittmachers, bei seiner Implantation oder
bei bestimmten Kontrollen. Weiterhin kann beispielsweise
bei einem programmierbaren  Herzschrittmacher auch die
Identität desjenigen, der die Parameter ändert, und
der Zeitpunkt der Änderung abgespeichert werden. Auch
die Patientendaten und gewisse Diagnosefakten können
abgespeichert werden.

Wenn die anfallende Datenmenge zu groß ist, kann vorteilhaft ein Teil dieser Daten in dem Speicher 13 des
Peripheriegerätes 10 gespeichert werden. Benutzt man
dabei beispielsweise die im Speicher 3 gelagerte Seriennummer des Herzschrittmachers 1 als Referenzadresse,
so können die administrativen Daten automatisch in dem
Speicher 13 des Peripheriegerätes 10 gespeichert und
bei Bedarf abgerufen werden. Größe und Art der verwendeten Speicher richtetn sich dabei nach dem erforderlichen
Speicherbedarf. Der Herzschrittmacher 1 bildet somit
zusammen mit dem Peripheriegerät 10 eine Einheit, die

0049812

dem behandelndenArzt sämtliche Daten für die Beurteilung der Herzschrittmsacherfunktion und die Wirksamkeit auf den zu behandelnden Patienten liefern kann. Diese Einheit befindet sich dann praktisch immer bei dem Patienten, um den es geht. Karteikarten, Berichte, und Datenblätter, die ansonsten häufig an unterschiedlichsten Stellen abgelegt sind, entfallen damit praktisch vollständig.

Fig. 2 zeigt in der gleichen schematischen Darstellungsform eines Blockschaltbildes eine erweiterte Ausführungsform. Gleiche Teile sind mit gleichen Bezugszeichen versehen.

Der Herzschrittmacher gemäß dem Ausführungsbeispiel nach Fig. 2 ist mit 9 bezeichnet. Als zusätzliche Elemente kommen ein Meßwerterfassungsglied 7 und unter Umständen ein Datenaufbereitungsglied 8 hinzu. Das Peripheriegerät 10 bleibt unverändert. Zusätzlich kommt ein weiteres Peripheriegerät 20 mit einem zusätzlichen Speicher 23, beispielsweise einem miniaturisierten Bandgerät für eine Langzeitregistrierung von Daten hinzu. Dieses zusätzliche Peripheriegerät enthält weiterhin eine Steuereinheit 24 und Mittel 26 zum Empfangen oder Übertragen der Daten.

Mit Hilfe des Meßwerterfassungsgliedes 7 können Daten eingesammelt werden, z. B. über das Betriebsverhalten des Herzschrittmachersystems und der Elektroden, über die Reaktion des Patienten auf die Herzschrittmacherbehandlung oder auch über den allgemeinen Zustand des Patienten, also rein diagnostische Daten. So können z. B. der Stromverbrauch des Herzschrittmachers, die Anzahl der abgegebenen Stimulierungsimpulse, der Herzrhythmus, die Amplituden der elektrischen Aktivitäten des Herzens, ein intrakardiales EKG, die Reizschwelle, Störungen durch Muskelpotentiale oder elektromagnetische Felder erfaßt werden. Weiter können auch andere diagnostische Daten, die den Zustand des Patienten betreffen, wie beispielsweise die geförderte Blutmenge des Herzens, der Sauerstoffgehalt des Blutes oder andere physiologische Parameter erfaßt werden. Wenn die anfallende Datenmenge zu groß wird oder ein unpassendes Format annimmt, kann durch das Datenaufbereitungsglied 8 eine Verdichtung der Daten oder eine Vorauswertung vorgenommen werden. Eine Datenverdichtung kann beispielsweise durch eine statistische Bearbeitung der gesammelten Daten geschehen, indem festgestellt wird, ob ein bestimmtes Ereignis eingetreten ist und wenn ja, wie oft. Es können auch Histogramme erstellt werden, die von einem Parameter abhängen.

Die durch das Meßwerterfassungsglied 8 eingesammelten Daten werden entweder direkt oder nach einer Verarbeitung in dem Datenaufbereitungsglied 8 mit Hilfe der Steuereinheit 4 in den Speicher 3 oder auch den Speicher 13 eingegeben.

Weiterhin ist bei diesem Ausführungsbeispiel vorgesehen, daß die Funktionen der Steuereinheiten oder des Datenaufbereitungsgliedes und auch die Inhalte der verschiedenen Speicher mit Hilfe des Peripheriegerätes 10 oder aber auch durch das Auftreten gewisser Ereignisse verändert werden können. Ein solches Ereignis kann z. B. ein Herzblock sein.

In gewissen Fällen kann es vorkommen, daß die erforderliche Datenmenge so groß wird, daß selbst der zusätzliche Speicher 13 des Peripheriegeräts 10 noch nicht ausreicht oder es aus Kostengründen oder anderen praktischen Erwägungen nicht vorteilhaft erscheint, die vorhandenen Speicher im Herzschrittmacher und in dem Peripheriegerät so groß zu machen. Für diesen Fall ist bei dem Ausführungsbeispiel gemäß der Figur 2 ein weiteres Peripheriegerät 20 vorgesehen, mit der das Meßwerterfassungsglied 7 und das Datenaufbereitungsglied 8
praktisch kontinuierlich kommunizieren können. Das
Peripheriegerät 20 enthält dazu als weiteren Speicher
23 z. B. ein miniaturisiertes Bandgerät, mit dem eine
Langzeitregistrierung von Meßwerten möglich ist.

Die in den Figuren zu den beiden Ausführungsbeispielen
in einzelnen Blöcken dargestellten Glieder können in
ihrer Gesamtheit oder auch nur teilweise mit den anderen
Gliedern der übrigen Herzschrittmacherfunktionen mit
hilfe der Mikroprozessortechnik realisiert werden. Die
Funktionen der dargestellten einzelnen Glieder wären
dann nicht mehr notwendigerweise physikalisch separierbar.

.2 Figuren
10 Patentansprüche

Patentansprüche

1. Herzschrittmacher, insbesondere implantierbarer, g e k e n n z e i c h n e t   d u r c h   einen Speicher (3) für Daten, die nicht direkt mit der Funktion des Herzschrittmachers (1) zu tun haben, Mittel (6) zum kontaktlosen Empfangen und/oder Überführen von Daten an bzw. von einer äußeren Apparatur (Peripheriegerät 10) sowie eine Steuereinheit (4) für den Datenfluß.

2. Herzschrittmacher nach Anspruch 1, d a d u r c h g e k e n n z e i c h n e t , daß der Speicher (3) zur Aufnahme administrativer und/oder diagnostischer Daten dient.

3. Herzschrittmacher nach Anspruch 1 oder 2, d a d u r c h g e k e n n z e i c h n e t , daß ein Meßwerterfassungsglied (7) vorgesehen ist, von dem aus die Daten gesteuert über die Steuereinheit (4) vorgewählten Plätzen des Speichers (3) zuführbar sind.

4. Herzschrittmacher nach Anspruch 3, d a d u r c h g e k e n n z e i c h n e t , daß ein Datenaufbereitungsglied (8) zur Vorauswertung und/oder Verdichtung der im Meßwerterfassungsglied (7) anfallenden Daten vorgesehen ist.

5. Herzschrittmacher nach einem der Ansprüche 1 bis 4, d a d u r c h g e k e n n z e i c h n e t , daß ein Zeitgeber (5) vorgeehen ist, durch den bestimmte Zeiten für den Datentransport festlegbar sind.

6. Herzschrittmacher nach einem der Ansprüche 1 bis 5, d a d u r c h g e k e n n z e i c h n e t , daß das Peripheriegerät (10) eine Datenausgabeeinheit (11) und/oder eine Dateneingabeeinheit (12), Mittel (16) zum Empfangen und/oder Überführen von Daten sowie eine Steuereinheit (14) für den Datenfluß enthält.

7. Herzschrittmacher nach einem der Ansprüche 1 bis 6, d a d u r c h   g e k e n n z e i c h n e t ,   daß das Peripheriegerät (10) einen zusätzlichen Speicher (13) enthält.

8. Herzschrittmacher nach einem der Ansprüche 1 bis 7,   d a d u r c h   g e k e n n z e i c h n e t , daß die Überführung der Daten zwischen Herzschrittmacher (1) und Peripheriegerät (10) in an sich bekannter Weise mittels Fernmessung erfolgt.

9. Herzschrittmacher nach einem der Ansprüche 1 bis 8, d a d u r c h   g e k e n n z e i c h n e t ,   daß ein weiteres Peripheriegerät (20) mit einem weiteren Speicher (23), Mittel (26) zum Empfangen oder Überführen von Daten und ebenfalls einer Steuereinheit (24) vorgesehen ist.

10. Herzschrittmacher nach Anspruch 9,   d a d u r c h   g e k e n n z e i c h n e t ,   daß als Speicher (23) ein miniaturisiertes Bandgerät vorgesehen ist.

FIG 1

FIG 2

**0049812**

Europäisches Patentamt

**EUROPÄISCHER RECHERCHENBERICHT**

Nummer der Anmeldung

EP 81 10 7745

## EINSCHLÄGIGE DOKUMENTE

| Kategorie | Kennzeichnung des Dokuments mit Angabe. soweit erforderlich. der maßgeblichen Teile | betrifft Anspruch |
|---|---|---|
| X | <u>FR - A - 2 379 104</u> (PACESETTER)<br><br>  * Seite 12, Zeilen 7-32; Seite 42, Zeilen 18-38; Seite 49, Zeilen 23-39; Seite 46, Zeilen 11-33; Seite 50, Zeilen 22-28; Seite 52; Seite 53; Seite 54, Zeilen 1-13 *<br><br>& DE - A - 2 803 366<br><br>-- | 1-3,6-9 |
| X | <u>GB - A - 2 026 870</u> (MEDTRONIC)<br><br>  * Seite 4, Zeilen 25-27; Seite 18, Zeilen 43-56 *<br><br>& DE - A - 2 929 498<br><br>-- | 1,2,5,6,8 |
| X | <u>US - A - 4 223 679</u> (PACESETTER)<br><br>  * Spalte 1, Zeilen 13-20; Spalte 2, Zeilen 41-55; Spalte 3, Zeilen 4-18 *<br><br>-- | 1-3,6,8 |
|  | <u>US - A - 4 223 678</u> (MIROWSKI)<br><br>  * Spalte 2, Zeile 23 - Spalte 3, Zeile 21 *<br><br>-- | 1,2,4-8,10 |
|  | <u>US - A - 4 142 533</u> (RESEARCH)<br><br>  * Spalte 3, Zeile 30 - Spalte 15, Zeile 5 *<br><br>-- | 2,6-8,10 |
| EP | <u>GB - A - 2 060 174</u> (MEDRAD)<br><br>  * Seite 1, Zeilen 53-62; 107-121; Seite 3, Zeilen 73-96; Seite 8, Zeilen 61-113 *<br><br>./. | 1-8 |

**KLASSIFIKATION DER ANMELDUNG** (Int. Cl.³)

A 61 N 1/08<br>1/36

**RECHERCHIERTE SACHGEBIETE** (Int. Cl.³)

A 61 N 1/08<br>1/36

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Grunden angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 20-01-1982 | SIMON |

EPA form 1503.1   06.78

Europäisches
Patentamt

**EUROPÄISCHER RECHERCHENBERICHT**

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | & DE - A - 3 035 732 | | |
| | -- | | |
| EP | WO - A - 80/2498 (MEDTRONIC) | 1-3 | |
| | * Seite 2, Zeilen 11-14; Seite 3, Zeile 9 - Seite 4, Zeile 15 * | | |
| | & EP - A - 0 028 640 | | |
| | ---- | | RECHERCHIERTE SACHGEBIETE (Int. Cl.³) |